# FASCICULE DE BREVET EUROPEEN

(11) **EP 1 172 088 B1**
(45) Date de publication et mention de la délivrance du brevet: **06.05.2004**
(21) Numéro de dépôt: 01401490.6
(22) Date de dépôt: 08.06.2001
(51) Int. Cl.: A61K 7/48, A61K 7/50

(54) **Composition cosmétique exothermique**
Exothermische kosmetische Zusammensetzung
Exothermic cosmetic composition

(30) Priorité: 11.07.2000 FR 0009062
(43) Date de publication de la demande: 16.01.2002
(73) Titulaire: L'OREAL, 75008 Paris (FR)
(72) Inventeur: Verdrel-Lahaxe, Delphine, 92160 Antony (FR); Bui-Bertrand, Liên, 91600 Savigny Sur Orge (FR)
(74) Mandataire: Rasson, Catherine

(56) Documents cités:
- EP-A- 0 897 719
- EP-A- 0 950 400
- EP-A- 1 051 964
- WO-A-93/08793
- GB-A- 1 344 043
- PATENT ABSTRACTS OF JAPAN vol. 2000, no. 01, 31 janvier 2000 (2000-01-31) & JP 11 292730 A (KAO CORP), 26 octobre 1999 (1999-10-26) & JP 11 292730 A (KAO CORP) 26 octobre 1999 (1999-10-26)
- DATABASE WPI Week 199919, 2 mars 1999 (1999-03-02) Derwent Publications Ltd., London, GB; AN 1999-226073 XP002225676 KIMURA MITSUTOSHI: "Skin cosmetic" & JP 11 060460 A (KAO CORP), 2 mars 1999 (1999-03-02) & PATENT ABSTRACTS OF JAPAN vol. 1999, no. 08, 30 juin 1999 (1999-06-30) & JP 11 060460 A (KAO CORP), 2 mars 1999 (1999-03-02)
- DATABASE WPI Week 199642, 13 août 1996 (1996-08-13) Derwent Publications Ltd., London, GB; AN 1996421886 XP002225677 KAWAKITA RYUSHI ET AL.: "Liquid cleaner" & JP 08 208455 A (KANSAI KOSO KK), 13 août 1996 (1996-08-13) & PATENT ABSTRACTS OF JAPAN vol. 1996, no. 12, 26 décembre 1996 (1996-12-26) & JP 08 208455 A (KANSAI KOUSO KK), 13 août 1996 (1996-08-13)

## Description

La présente invention a pour objet une composition cosmétique exothermique, comprenant une zéolite, un tensioactif et un halogénure de magnésium ou de calcium, et les utilisations de ladite composition notamment pour le nettoyage et/ou le démaquillage de la peau et/ou des muqueuses. L'invention se rapporte aussi à un procédé cosmétique de nettoyage et/ ou de démaquillage de la peau à l'aide de ladite composition.

Il est connu d'utiliser des compositions cosmétiques anhydres exothermiques, c'est-à-dire ayant un effet chauffant lors de l'application sur la peau. La propriété exothermique de ces compositions est généralement apportée par la présence d'un composé exothermique tel que les zéolites et les polyols comme la glycérine ou les polyéthylène glycols. Ces compositions sont particulièrement appropriées pour le nettoyage de la peau. Ainsi, le document EP-A-974340 décrit une composition chauffante à base de polyols, dont les propriétés d'étalement et de rinçage sont améliorées par la présence d'oxyde d'aluminium.

Les zéolites sont particulièrement efficaces comme composés exothermiques. Toutefois, pour que les compositions exothermiques les contenant aient de bonnes propriétés de nettoyage, on est amené à y ajouter des tensioactifs, et notamment des tensioactifs moussants. Ainsi, le document EP-A-897719 décrit une composition de nettoyage de la peau comprenant un composé générant de la chaleur, et en particulier une zéolite, associé à un tensioactif anionique. Or, l'addition de tensioactifs, et notamment de tensioactifs non ioniques, dans ces compositions entraînent une instabilité de la composition, qui se traduit par une hétérogénéité de la composition. Les zéolites ont alors tendance à migrer vers le fond, provoquant une décantation dans la composition. Une telle instabilité rend rédhibitoire l'utilisation de la composition.

Pour améliorer la stabilité de ces compositions, on a pensé à y ajouter des polymères épaississants tels que les carbopol. Toutefois, l'addition de ces polymères présente l'inconvénient d'aboutir à des gels collants et filants qui, en outre, ne sont pas homogènes.

Aussi, il subsiste le besoin d'une composition cosmétique exothermique nettoyante stable dans le temps.

La demanderesse a trouvé de manière surprenante que l'addition d'halogénure de magnésium ou de calcium dans une composition exothermique contenant une zéolite et un tensioactif et notamment un tensioactif non ionique, permettait d'obtenir une composition nettoyante stable.

Ainsi, l'invention se rapporte à une composition cosmétique exothermique, comprenant dans un milieu anhydre physiologiquement acceptable, au moins une zéolite, au moins un tensioactif et au moins un halogénure de magnésium ou de calcium.

On entend ici par « composition exothermique » une composition telle que l'utilisateur ressente un échauffement lors de l'application de la composition sur la peau. C'est une composition dont la température en présence d'eau (l'eau ajoutée lors de l'utilisation ou bien l'eau présente dans la peau) peut s'élever de plusieurs degrés (un à vingt degrés) de manière instantanée. Cet effet chauffant va permettre l'ouverture des pores de la peau et donc un meilleur nettoyage de la peau.

La composition selon l'invention est constituée d'un milieu anhydre. On entend ici par « anhydre » un milieu quasiment anhydre, c'est-à-dire comprenant généralement moins de 6 % en poids d'eau, de préférence moins de 4 % en poids d'eau, et encore mieux moins de 1 % en poids d'eau par rapport au poids total de la composition. Elle peut être aussi totalement anhydre.

La composition de l'invention étant une composition cosmétique et donc destinée à une application topique, elle comporte un milieu physiologiquement acceptable, c'est-à-dire compatible avec la peau, les muqueuses et/ou les fibres kératiniques.

La composition de l'invention est homogène et stable dans le temps. Elle se présente généralement sous forme d'un gel translucide à opaque, mais elle peut se présenter aussi sous forme de crème, de pâte et même de poudre si tous les constituants utilisés sont poudreux, ladite poudre pouvant être utilisée telle quelle ou dans une éponge ou bien encore imprégnée sur une lingette. La composition de l'invention peut, quelle que soit sa forme, être utilisée sur une lingette.

La composition obtenue est stable et a une viscosité satisfaisante, sans qu'il soit besoin d'ajouter de gélifiant et notamment de polymère gélifiant. Selon un mode préféré de réalisation de l'invention, la composition est exempte de polymère soluble dans l'eau.

L'halogénure de magnésium ou de calcium a l'avantage non seulement de permettre d'obtenir une composition stable, mais aussi de permettre d'ajuster le pH de la composition à une valeur appropriée pour une utilisation topique, car les zéolites confèrent aux compositions les contenant un pH basique élevé peu compatible avec une utilisation topique sur la peau. Le pH obtenu pour la composition selon l'invention va alors généralement de 5 à 9.

La présente invention a aussi pour objet l'utilisation d'au moins un halogénure de magnésium ou de calcium, pour stabiliser une composition contenant au moins une zéolithe et au moins un tensioactif.

L'halogénure de magnésium ou de calcium peut être choisi en particulier parmi les iodures, chlorures et bromures de calcium ou de magnésium et leurs mélanges. Selon un mode préféré de réalisation de l'invention, l'halogénure de métal alcalino-terreux est le chlorure de calcium.

La quantité d'halogénure(s) de magnésium ou de calcium peut aller par exemple de 1 à 15 % en poids et de préférence de 2 à 10 % en poids par rapport au poids total de la composition.

Les zéolites sont des silicoaluminates. A titre de zéolites, on peut citer notamment les zéolites activées, et par exemple les zéolites A, les zéolites X comme celles commercialisées par les sociétés Fluka et Union Carbide, les zéolites MAP telles que décrites dans le document EP-A-384070, les zéolites A activées. Les cations présents dans les zéolites utilisées comprennent notamment Na, K, Ca, Zn, Mg, Li, Cu et leurs combinaisons. On peut notamment utiliser comme zéolite activée, les zéolites A-3, A-4 et A-5 de formule :

MₓO-Al₂O₃-ySiO₂-zH₂O

dans laquelle M est un métal alcalin ou alcalino-terreux, et x, y et z sont indépendamment les uns des autres un nombre quelconque. Ces zéolites se présentent sous forme de particules ayant de préférence une granulométrie allant de 3 à 6 microns.

Ces zéolites et leur procédé de préparation sont décrits dans le document EP-A-187912 incorporé ici pour référence. On peut utiliser notamment celles commercialisées sous le nom de ADVERA 401N ET ADVERA 402N par la société PQ Corporation.

On peut additionner à la zéolite ou aux zéolites utilisées dans la composition de l'invention, tout autre agent exothermique, donc susceptible de dégager de la chaleur lors de l'hydratation de la composition, et en particulier un ou plusieurs polyols.

A titre de polyol, on peut citer notamment les polyols ayant au moins 2 groupes hydroxyle et au moins 3 atomes de carbone, et par exemple la glycérine, la diglycérine, le propylène glycol, le dipropylène glycol, le butylène glycol, l'hexylène glycol, le polyéthylène glycol et les polyéthylène glycols de poids moléculaire inférieur à 600 comme le PEG-8, les sucres tels que le sorbitol, et leurs mélanges. Comme polyols, on utilise de préférence la glycérine, le butylène glycol, le propylène glycol, le dipropylène glycol, le PEG-8 et leurs mélanges.

Selon un mode préféré de réalisation de l'invention, la composition contient une zéolite ou un mélange de zéolites, et de préférence un mélange de zéolite(s) et de polyol(s) et plus particulièrement un mélange de zéolite activée et d'un ou plusieurs polyols choisi parmi le butylène glycol, le propylène glycol, la glycérine, le dipropylène glycol et/ou le PEG-8.

La quantité d'agent exothermique (zéolite(s) ou de zéolite(s) additionné(s) de polyols), doit être suffisante pour que la composition soit exothermique et que, par conséquent, l'utilisateur ressente effectivement un échauffement lors de l'application de la composition sur la peau. En pratique, la quantité de zéolite(s) va généralement de 5 à 95 % en poids, de préférence de 10 à 70 % en poids et mieux de 20 à 60 % en poids par rapport au poids total de la composition. La quantité de polyol(s) peut aller par exemple de 20 à 90 %, de préférence de 30 à 90 % en poids et mieux de 40 à 90 % en poids par rapport au poids total de la composition.

La composition selon l'invention contient au moins un tensioactif qui est généralement un tensioactif nettoyant et/ou moussant, et qui peut être choisi parmi les tensioactifs non ioniques, les tensioactifs anioniques, les tensioactifs amphotères et leurs mélanges. La quantité de tensioactif(s) peut aller de 0,5 à 20 % en poids de matière active, de préférence de 1 à 15 % en poids et mieux de 2 à 10 % en poids de matière active par rapport au poids total de la composition. Les tensioactifs peuvent se présenter sous forme de poudre, de pâte ou de liquide. Ils peuvent aussi être en dispersion aqueuse dans la mesure où la quantité d'eau n'est pas trop importante et ne nuit pas aux qualités exothermiques de la composition.

De façon avantageuse, le ou les tensioactifs sont sous forme d'une pâte.

Comme tensioactifs non ioniques utilisables dans l'invention, on peut citer par exemple les condensats d'oxydes d'alkylène et d'alkylphénols comme l'octyl phénol éthoxylé tel que celui commercialisé sous le nom Triton X45 par la société Rohm & Haas ; les alkylpolyglucosides ; les éthers d'alcool gras et de polyols comme par exemple le Polyglyceryl-3 hydroxylauryl Ether (nom CTFA) commercialisé sous la dénomination Chimexane NF par la Société Chimex ; les dérivés non ioniques du glucose et du méthyl glucose, comportant des groupements oxyde de polyéthylène ou oxyde de polypropylène, ces dérivés pouvant ou non comporter une chaîne grasse en C8 à C30, tels que le di-oléate de méthylglucose oxyéthyléné (120 OE) (nom CTFA: PEG-120 methylglucose dioleate) commercialisé sous la dénomination Glucamate DOE 120 par la Société Amerchol ou le méthylglucose oxypropoxylé (20 OP) vendu sous la dénomination commerciale Glucam E 20 par la Société Amerchol ; les amides gras oxyéthylénés tels que le PEG-5 cocamide, et les mélanges de ces tensioactifs.

Comme tensioactifs anioniques, on peut citer par exemple les polyalkylènes glycols éther d'alcools gras ; les taurates ; les acyl lactylates comme le stéaroyl lactylate de sodium (par exemple Pationic SSL commercialisé par la société Maprecos) ; les alkyl sulfates comme le lauryl sulfate de sodium (Sipon LCS commercialisé par la société Henkel) ; les alkyl sulfates de glycéryle comme le cocoglycéryl sulfate de sodium (commercialisé par la société Nikko sous le nom Nikkol SGC-80N) ; les alkyl sulfates polyoxyéthylénés ; les alkyl éthers sulfates comme le lauryl éther sulfate de monoéthanolamine; les alkyl éthers carboxylates ; les monoalkyl ou dialkyl phosphates comme le monohexyl-2-décyl phosphate d'arginine (MAP-16G-ARG commercialisé par la société Kao Chemicals); les alkyl phosphate éthoxylés ; les N-acylsarcosinates comme le myristoylsarcosinate de sodium (par exemple Nikkol Sarcosinate MN commercialisé par la société Nikko) ; les N-acylglutamates comme le lauroylglutamate de sodium (par exemple Amisoft LS11 commercialisé par la société Ajinomoto) ; les acyliséthionates comme le cocoyliséthionate de sodium commercialisé notamment par la société Jordan (Jordapon Cl); les succinamates ; les savons comme le laurate, myristate, palmitate ou stéarate de potassium ou de sodium ; et leurs mélanges.

Comme tensioactifs amphotères ou zwitterions, on peut citer par exemple les bétaïnes et les dérivés de bétaïnes ; les sultaïnes et les dérivés de sultaïnes ; les dérivés imidazolinium, comme le cocoamphodiacétate de disodium ; et leurs mélanges.

Selon un mode particulier de réalisation de l'invention, le tensioactif est un tensioactif non ionique, un mélange de tensioactifs non ioniques ou un mélange de tensioactifs non ionique et anionique.

La composition peut, en outre, contenir un ou plusieurs autres ingrédients notamment lipophiles, classiquement utilisés dans les compositions de nettoyage ou de démaquillage. Ces ingrédients sont, notamment, des huiles, des parfums, des conservateurs, des antioxydants, des séquestrants, des charges, des colorants, des actifs cosmétiques ou dermatologiques, ou leurs mélanges. Ces adjuvants sont utilisés dans les proportions habituelles des compositions nettoyantes et/ou de soin, et par exemple de 0,01 à 10 % du poids total de la composition. Ces adjuvants doivent être de nature et utilisés en quantité telles qu'ils ne perturbent pas les propriétés recherchées pour la composition de l'invention.

Comme charges, on peut citer par exemple le talc, l'amidon modifié ou non, et notamment les amidons estérifiés par l'anhydride octénylsuccinique et plus particulièrement le "Aluminium Starch octenyl succinate" tel que le produit commercialisé par la société National Starch sous le nom de Dry-Flo.

Comme actifs utilisables dans l'invention, on peut citer des antibactériens comme l'octopirox et le triclosan, des agents kératolytiques comme l'acide salicylique, des huiles essentielles, des vitamines et en particulier la niacinamide (vitamine PP) et le panthénol (vitamine B3).

La composition de l'invention est particulièrement adaptée pour le nettoyage et/ou le démaquillage de la peau et/ou des muqueuses et plus particulièrement pour le nettoyage et/ou le démaquillage de la peau.

L'invention a encore pour objet l'utilisation cosmétique de la composition telle que définie ci-dessus, pour le nettoyage et/ou le démaquillage de la peau et/ou des muqueuses.

La composition de l'invention se présente généralement sous forme d'un gel épais. On peut utiliser ce gel de 2 façons :
1) soit on humidifie légèrement le visage, puis on applique le gel sur le visage : la réaction exothermique se produit alors. On fait mousser ensuite en rajoutant de l'eau, puis on rince.
2) soit on applique le gel sur peau sèche par massage ; la réaction exothermique démarre alors grâce à l'eau d'hydratation de la peau, puis on ajoute un peu d'eau pour accroître la réaction exothermique, on fait mousser puis on rince.

Ainsi, l'invention a encore pour objet un procédé cosmétique de démaquillage et/ou de nettoyage de la peau, consistant à humidifier légèrement la peau, et notamment la peau du visage, à appliquer sur la peau la composition telle que définie ci-dessus, à faire mousser la composition en rajoutant de l'eau, et enfin à rincer la peau.

L'invention a aussi pour objet un procédé cosmétique de démaquillage et/ou de nettoyage de la peau, consistant à appliquer sur peau sèche par massage, la composition telle que définie ci-dessus, à ajouter un peu d'eau, à faire mousser la composition puis à rincer la peau.

Comme indiqué plus haut, la composition de l'invention peut être utilisée sur un substrat tel qu'une éponge ou une lingette, et constituer un article de nettoyage à un seul usage.

L'invention a aussi pour objet un article de nettoyage comprenant un substrat insoluble dans l'eau et une composition telle que définie ci-dessus. Le substrat insoluble dans l'eau peut être une éponge ou une lingette, par exemple une lingette en produit non tissé, à une ou plusieurs couches, à l'état humide ou sec.

Les exemples ci-après sont donnés à titre illustratif et non limitatif en vue de mieux faire ressortir les caractéristiques de l'invention. Les quantités y sont données en % en poids.

### Exemple 1 : Composition nettoyante chauffante

- Butylène glycol 18 %
- PEG-8 18 %
- Glycérine 14 %
- Polyglycéryl-3hydroxylauryl ether 6 %
- PEG-120 méthyl glucose dioléate 1 %
- Zéolite (aluminosilicate de potassium activée : ADVERA 402 N) 40 %
- Chlorure de calcium 3 %

Mode opératoire : On dissout le chlorure de calcium dans le PEG-8 et le butylène glycol. Puis on ajoute les tensioactifs fondus dans la glycérine à 65°C. On homogénéise ces 2 phases, on refroidit le mélange à température ambiante puis on ajoute la zéolite en homogénéisant.

Le produit obtenu est un gel blanc stable, d'application et d'élimination faciles, doux au toucher et ayant un bon pouvoir nettoyant.

**Exemple 1 comparatif :** on a reproduit l'exemple 1 mais sans chlorure de calcium. On a observé une instabilité de la composition avec décantation de la zéolite vers le bas et décantation des glycols vers le haut.

### Exemple 2 : Composition nettoyante chauffante

- Butylène glycol 25 %
- PEG-8 25 %
- Glycérine 12 %
- PEG-5 cocamide 6 %
- PEG-120 méthyl glucose dioléate 2 %
- Zéolite (aluminosilicate de potassium activée : ADVERA 402 N) 20 %
- Chlorure de calcium 10 %

Le mode opératoire est identique à celui de l'exemple 1.

Le produit obtenu est un gel blanc stable, d'application et d'élimination faciles, doux au toucher et ayant un bon pouvoir nettoyant.

### Exemples 3 et 4 et exemples comparatifs 2 et 3 :

| Composition | Exemple 3 selon l'invention | Exemple 4 selon l'invention | Exemple comparatif 2 | Exemple comparatif 3 |
|---|---|---|---|---|
| PEG-8 | 14.3 | 14.3 | 14.3 | 14.3 |
| Butylène glycol | 14.3 | 14.3 | 14.3 | 14.3 |
| CaCl2,2 H20 | 3 | 0 | 0 | 0 |
| NaCI | 0 | 0 | 3 | 0 |
| MgCl2,6 H20 | 0 | 3 | 0 | 0 |
| Diméthicone copolyol | 2 | 2 | 2 | 2 |
| Glycérine | 27 | 24 | 24 | 24 |
| PEG-120 méthyl glucose dioléate | 1 | 1 | 1 | 1 |
| Zéolites | 30 | 30 | 30 | 30 |
| Charges diverses : Kaolin + oxyde de titane + nacres | 11.4 | 11.4 | 11.4 | 11.4 |
| Viscosité en Poises | 255 | 170 | Non mesurée car le chlorure de sodium reste insoluble | 88 |

L'exemple comparatif 3 sans sel a une viscosité nettement inférieure aux exemples selon l'invention. Ce tableau montre que les sels de calcium et de magnésium permettent bien de stabiliser la composition et d'obtenir une bonne viscosité sans utilisation de polymère gélifiant.

Par ailleurs, dans les exemples selon l'invention, le sel de magnésium ou de calcium est dissous dans les polyols. En revanche, dans l'exemple comparatif 2, le sel (chlorure de sodium) reste insoluble, et le but recherché n'est donc pas atteint. Ainsi, l'utilisation de NaCI ne nous permet pas de stabiliser la composition.

## Revendications

1. Composition cosmétique exothermique, comprenant dans un milieu anhydre physiologiquement acceptable, au moins une zéolite, au moins un tensioactif et au moins un halogénure de magnésium ou de calcium.

2. Composition selon la revendication 1, **caractérisée en ce que** l'halogénure de magnésium ou de calcium est choisi parmi les iodures, chlorures et bromures de calcium ou de magnésium et leurs mélanges.

3. Composition selon la revendication 1 ou 2, **caractérisée en ce que** l'halogénure est le chlorure de calcium.

4. Composition selon l'une quelconque des revendications précédentes, **caractérisée en ce que** la quantité d'halogénure(s) de magnésium ou de calcium va de 1 à 15 % en poids par rapport au poids total de la composition.

5. Composition selon l'une quelconque des revendications précédentes, **caractérisée en ce que** la zéolite est une zéolite activée choisie parmi les zéolites A, les zéolites X, les zéolites MAP.

6. Composition selon l'une quelconque des revendications précédentes, **caractérisée en ce que** la quantité de zéolite(s) va de 5 à 95 % en poids par rapport au poids total de la composition.

7. Composition selon l'une quelconque des revendications précédentes, **caractérisée en ce qu'**elle contient en outre au moins un polyol.

8. Composition selon la revendication précédente, **caractérisée en ce que** le polyol est choisi parmi la glycérine, la diglycérine, le propylène glycol, le dipropylène glycol, le butylène glycol, l'hexylène glycol, le polyéthylène glycol et les polyéthylène glycols de poids moléculaire inférieur à 600, les sucres et leurs mélanges.

9. Composition selon l'une des revendications 7 ou 8 **caractérisée en ce que** la quantité de polyol(s) va de 20 à 90 % en poids par rapport au poids total de la composition.

10. Composition selon l'une quelconque des revendications précédentes, **caractérisée en ce que** la quantité de zéolite(s) ou la quantité de zéolithe(s) et polyol(s) est suffisante pour que la composition soit exothermique.

11. Composition selon l'une quelconque des revendications précédentes, **caractérisée en ce que** le tensioactif est un tensioactif nettoyant et/ou moussant, choisi parmi les tensioactifs non ioniques, les tensioactifs anioniques, les tensioactifs amphotères et leurs mélanges.

12. Composition selon la revendication précédente, **caractérisée en ce que** le tensioactif non ionique est choisi parmi les condensats d'oxydes d'alkylène et d'alkylphénols, les alkylpolyglucosides, les éthers d'alcool gras et de polyols, les dérivés non ioniques du glucose et du méthyl glucose, comportant des groupements oxyde de polyéthylène ou oxyde de polypropylène, et leurs mélanges.

13. Composition selon la revendication 11, **caractérisée en ce que** le tensioactif anionique est choisi parmi les polyalkylènes glycols éther d'alcools gras, les taurates, les acyl lactylates, les alkyl sulfates, les alkyl sulfates de glycéryle, les alkyl sulfates polyoxyéthylénés, les alkyl éthers sulfates, les alkyl éthers carboxylates, les monoalkyl ou dialkyl phosphates, les alkyl phosphate éthoxylés, les N-acylsarcosinates, les N-acylglutamates, les acyliséthionates, les succinamates, les savons et leurs mélanges.

14. Composition selon l'une quelconque des revendications précédentes, **caractérisée en ce que** la quantité de tensioactif(s) va de 0,5 à 20 % en poids par rapport au poids total de la composition.

15. Composition selon l'une quelconque des revendications précédentes, **caractérisée en ce qu'**elle se présente sous forme d'un gel, d'une crème, d'une pâte ou d'une poudre.

16. Utilisation cosmétique de la composition selon l'une des revendications précédentes pour le nettoyage et/ou le démaquillage de la peau et/ou des muqueuses.

17. Procédé cosmétique de démaquillage et/ou de nettoyage de la peau, consistant à humidifier légèrement la peau, à appliquer sur la peau la composition selon l'une quelconque des revendications 1 à 15, à faire mousser la composition en rajoutant de l'eau et à rincer la peau.

18. Procédé cosmétique de démaquillage et/ou de nettoyage de la peau, consistant à appliquer sur peau sèche par massage, la composition selon l'une quelconque des revendications 1 à 15, à ajouter un peu d'eau, à faire mousser la composition puis à rincer la peau.

19. Utilisation d'au moins un halogénure de magnésium ou de calcium, pour stabiliser une composition comprenant dans un milieu anhydre physiologiquement acceptable au moins une zéolithe et au moins un tensioactif.

20. Article de nettoyage comprenant un substrat insoluble dans l'eau et une composition selon l'une quelconque des revendications 1 à 15.

## Patentansprüche

1. Wärmeentwickelnde kosmetische Zusammensetzung, die in einem wasserfreien physiologisch akzeptablen Medium mindestens einen Zeolithen, mindestens einen grenzflächenaktiven Stoff und mindestens ein Magnesium- oder Calciumhalogenid enthält.

2. Zusammensetzung nach Anspruch 1, **dadurch gekennzeichnet, dass** das Magnesium- oder Calciumhalogenid unter den Iodiden, Chloriden und Bromiden von Calcium oder Magnesium und deren Gemischen ausgewählt ist.

3. Zusammensetzung nach Anspruch 1 oder 2, **dadurch gekennzeichnet, dass** das Halogenid das Calciumchlorid ist.

4. Zusammensetzung nach einem der vorhergehenden Ansprüche, **dadurch gekennzeichnet, dass** der Mengenanteil des Magnesium- oder Calciumhalogenids oder der Magnesium- oder Calciumhalogenide im Bereich von 1 bis 15 Gew.-%, bezogen auf das Gesamtgewicht der Zusammensetzung, liegt.

5. Zusammensetzung nach einem der vorhergehenden Ansprüche, **dadurch gekennzeichnet, dass** der Zeolith ein aktivierter Zeolith ist, der unter den Zeolithen A, Zeolithen X und Zeolithen MAP ausgewählt ist.

6. Zusammensetzung nach einem der vorhergehenden Ansprüche, **dadurch gekennzeichnet, dass** der Mengenanteil des oder der Zeolithe im Bereich von 5 bis 95 Gew.-%, bezogen auf das Gesamtgewicht der Zusammensetzung, liegt.

7. Zusammensetzung nach einem der vorhergehenden Ansprüche, **dadurch gekennzeichnet, dass** sie ferner mindestens ein Polyol enthält.

8. Zusammensetzung nach dem vorhergehenden Anspruch, **dadurch gekennzeichnet, dass** das Polyol unter Glycerin, Diglycerin, Propylenglykol, Dipropylenglykol, Butylenglykol, Hexylenglykol, Polyethylenglykol und den Polyethylenglykolen mit einer Molmasse unter 600, Zuckern und deren Gemischen ausgewählt ist.

9. Zusammensetzung nach Anspruch 7 oder 8, **dadurch gekennzeichnet, dass** der Mengenanteil des Polyols oder der Polyole im Bereich von 20 bis 90 Gew.-%, bezogen auf das Gesamtgewicht der Zusammensetzung, liegt.

10. Zusammensetzung nach einem der vorhergehenden Ansprüche, **dadurch gekennzeichnet, dass** der Mengenanteil des oder der Zeolithe oder der Mengenanteil des oder der Zeolithe und des oder der Polyole ausreichend ist, damit die Zusammensetzung Wärme entwickelt.

11. Zusammensetzung nach einem der vorhergehenden Ansprüche, **dadurch gekennzeichnet, dass** der grenzflächenaktive Stoff ein reinigender und/oder schäumender grenzflächenaktiver Stoff ist, der unter den nichtionischen Tensiden, anionischen Tensiden, amphoteren Tensiden und deren Gemischen ausgewählt ist.

12. Zusammensetzung nach dem vorhergehenden Anspruch, **dadurch gekennzeichnet, dass** das nichtionische Tensid unter den Kondensaten von Alkylenoxiden und Alkylphenolen, Alkylpolyglycosiden, Polyolfettalkoholethern, nichtionischen Glucosederivaten und Methylglucosederivaten, die Polyethylenoxid oder Polypropylenoxidgruppen enthalten, und deren Gemischen ausgewählt ist.

13. Zusammensetzung nach Anspruch 11, **dadurch gekennzeichnet, dass** das anionische Tensid unter den Fettalkoholpolyalkylenglykolethern, Tauraten, Acyllactylaten, Alkylsulfaten, Glycerylalkylsulfaten, polyethoxylierten Alkylsulfaten, Alkylethersulfaten, Alkylethercarboxylaten, Monoalkyl- oder Dialkylphosphaten, ethoxylierten Alkylphosphaten, N-Acylsarcosinaten, N-Acylglutamaten, Acylisethionaten, Succinamaten, Seifen und deren Gemischen ausgewählt ist.

14. Zusammensetzung nach einem der vorhergehenden Ansprüche, **dadurch gekennzeichnet, dass** der Mengenanteil des Tensids oder der Tenside im Bereich von 0,5 bis 20 Gew.-%, bezogen auf das Gesamtgewicht der Zusammensetzung, liegt.

15. Zusammensetzung nach einem der vorhergehenden Ansprüche, **dadurch gekennzeichnet, dass** sie als Gel, Creme, Paste oder Pulver vorliegt.

16. Kosmetische Verwendung der Zusammensetzung nach einem der vorhergehenden Ansprüche zur Reinigung und/ oder zum Abschminken der Haut und/oder der Schleimhäute.

17. Kosmetisches Verfahren zum Abschminken und/oder zur Reinigung der Haut, das darin besteht, die Haut etwas zu befeuchten, auf die Haut eine Zusammensetzung nach einem der Ansprüche 1 bis 15 aufzutragen, die Zusammensetzung durch Wasserzugabe zum Schäumen zu bringen und die Haut zu spülen.

18. Kosmetisches Verfahren zum Abschminken und/oder zur Reinigung der Haut, das darin besteht, auf die trockene Haut durch Massieren eine Zusammensetzung nach einem der Ansprüche 1 bis 15 aufzutragen, etwas Wasser zuzufügen, die Zusammensetzung zum Schäumen zu bringen und anschließend die Haut zu spülen.

19. Verwendung mindestens eines Magnesium- oder Calciumhalogenids zur Stabilisierung einer Zusammensetzung, die in einem wasserfreien, physiologisch akzeptablen Medium mindestens einen Zeolithen und mindestens einen grenzflächenaktiven Stoff enthält.

20. Reinigungsartikel, der ein in Wasser unlösliches Substrat und eine Zusammensetzung nach einem der Ansprüche 1 bis 15 umfasst.

## Claims

1. Exothermic cosmetic composition comprising, in a physiologically acceptable anhydrous medium, at least one zeolite, at least one surfactant and at least one magnesium or calcium halide.

2. Composition according to Claim 1, **characterized in that** the magnesium or calcium halide is chosen from calcium or magnesium iodides, chlorides and bromides, and mixtures thereof.

3. Composition according to Claim 1 or 2, **characterized in that** the halide is calcium chloride.

4. Composition according to any one of the preceding claims, **characterized in that** the amount of magnesium or calcium halide(s) ranges from 1% to 15% by weight relative to the total weight of the composition.

5. Composition according to any one of the preceding claims, **characterized in that** the zeolite is an activated zeolite chosen from zeolites A, zeolites X and zeolites MAP.

6. Composition according to any one of the preceding claims, **characterized in that** the amount of zeolite(s) ranges from 5% to 95% by weight relative to the total weight of the composition.

7. Composition according to any one of the preceding claims, **characterized in that** it also contains at least one polyol.

8. Composition according to the preceding claim, **characterized in that** the polyol is chosen from glycerol, diglycerol, propylene glycol, dipropylene glycol, butylene glycol, hexylene glycol, polyethylene glycol and polyethylene glycols with a molecular weight of less than 600, and sugars, and mixtures thereof.

9. Composition according to one of Claims 7 or 8, **characterized in that** the amount of polyol(s) ranges from 20% to 90% by weight relative to the total weight of the composition.

10. Composition according to any one of the preceding claims, **characterized in that** the amount of zeolite(s) or the amount of zeolite(s) and polyol(s) is sufficient for the composition to be exothermic.

11. Composition according to any one of the preceding claims, **characterized in that** the surfactant is a cleansing and/or foaming surfactant chosen from nonionic surfactants, anionic surfactants and amphoteric surfactants, and mixtures thereof.

12. Composition according to the preceding claim, **characterized in that** the nonionic surfactant is chosen from condensates of alkylene oxides and of alkylphenols, alkylpolyglucosides, ethers of fatty alcohols and of polyols, nonionic derivatives of glucose and of methylglucose, comprising polyethylene oxide or polypropylene oxide groups, and mixtures thereof.

13. Composition according to Claim 11, **characterized in that** the anionic surfactant is chosen from polyalkylene glycol ethers of fatty alcohols, taurates, acyl lactylates, alkyl sulphates, glyceryl alkyl sulphates, polyoxyethylenated alkyl sulphates, alkyl ether sulphates, alkyl ether carboxylates, monoalkyl or dialkyl phosphates, ethoxylated alkyl phosphates, N-acylsarcosinates, N-acylglutamates, acylisethionates, succinamates and soaps, and mixtures thereof.

14. Composition according to any one of the preceding claims, **characterized in that** the amount of surfactant(s) ranges from 0.5% to 20% by weight relative to the total weight of the composition.

15. Composition according to any one of the preceding claims, **characterized in that** it is in the form of a gel, a cream, a paste or a powder.

16. Cosmetic use of the composition according to one of the preceding claims, for cleansing and/or removing make-up from the skin and/or mucous membranes.

17. Cosmetic process for removing make-up from and/or for cleansing the skin, which consists in slightly moistening the skin, in applying the composition according to any one of Claims 1 to 15 to the skin, in working the composition into a lather by adding water, and in rinsing the skin.

18. Cosmetic process for removing make-up from and/or for cleansing the skin, which consists in applying the composition according to any one of Claims 1 to 15 to dry skin by massaging, in adding a small amount of water, in working the composition into a lather and then in rinsing the skin.

19. Use of at least one calcium or magnesium halide to stabilize a composition comprising at least one zeolite and at least one surfactant in a physiologically acceptable anhydrous medium.

20. Cleansing article comprising a substrate which is insoluble in water and a composition according to any one of Claims 1 to 15.
